(19) Europäisches Patentamt
European Patent Office
Office européen des brevets

(11) Publication number: **0 118 046**
**A1**

(12) # EUROPEAN PATENT APPLICATION

(21) Application number: 84101232.1

(22) Date of filing: 07.02.84

(51) Int. Cl.[3]: C 07 C 69/76
C 07 C 69/753, C 07 C 69/616
C 09 K 3/34, G 02 F 1/13

(30) Priority: 07.02.83 JP 17518/83

(43) Date of publication of application:
12.09.84 Bulletin 84/37

(84) Designated Contracting States:
CH DE FR GB IT LI

(71) Applicant: HITACHI, LTD.
6, Kanda Surugadai 4-chome Chiyoda-ku
Tokyo 100(JP)

(71) Applicant: Kanto Chemical Co., Inc.
3-7, Nihonbashi
Chuo-ku Tokyo(JP)

(72) Inventor: Yokokura, Hisao
2-10-1, Higashi-narusawa-cho
Hitachi-shi Ibaraki-ken(JP)

(72) Inventor: Kitamura, Teruo
3600-234, Nakane
Katsuta-shi Ibaraki-ken(JP)

(72) Inventor: Okabe, Yoshiaki
2-1-12, Higashi-cho
Hitachi-shi Ibaraki-ken(JP)

(72) Inventor: Mukoo, Akio
498-21, Kasahara-cho
Mito-shi Ibaraki-ken(JP)

(72) Inventor: Abe, Hidetoshi
1293-5, Higashiishikawa
Katsuta-shi Ibaraki-ken(JP)

(72) Inventor: Fujii, Tsunenori
3231-7, Aoyagi-cho
Souka-shi Saitama-ken(JP)

(72) Inventor: Yoshida, Masahiro
3-23-4, Minami, Satte-cho
Kitakatsushika-gun Saitama-ken(JP)

(72) Inventor: Oomiri, Hideki
2090-820, Hokuya-cho
Souka-shi Saitama-ken(JP)

(72) Inventor: Okawa, Hisashi
2-5-4, Minami
Kasukabe-shi Saitama-ken(JP)

(72) Inventor: Kurihara, Makoto
1-10-16, Shinmei
Souka-shi Saitama-ken(JP)

(72) Inventor: Suzuki, Kenji
89, Oaza Ooeda
Kasukabe-shi Saitama-ken(JP)

(74) Representative: Patentanwälte Beetz sen. - Beetz jun.
Timpe - Siegfried - Schmitt-Fumian
Steinsdorfstrasse 10
D-8000 München 22(DE)

(54) **Liquid crystal compounds, liquid crystal compositions and liquid crystal display devices.**

(57) The liquid crystal ~~compound of the present~~ invention is characterized by having the following molecular structure:
(a) the molecular skeleton has two or more six-membered rings,
(b) the respective six-membered rings are bonded directly to each other,
(c) a carbon atom in each of the terminal six-membered rings is bonded directly to an acyclic terminal group,
(d) one of the acyclic terminal groups bonded to the carbon atom of the six-membered ring is an alkyl group or an alkoxycarbonyl-free group bonding through an alkylene group, and
(e) the other acyclic terminal group bonded to the carbon atom in the six-membered ring has a carbonyl group and a terminal alkoxyl group.

EP 0 118 046 A1

0118046

# TITLE OF THE INVENTION

LIQUID CRYSTAL COMPOUNDS, LIQUID CRYSTAL COMPOSITIONS AND LIQUID CRYSTAL DISPLAY DEVICES

## BACKGROUND OF THE INVENTION

### FIELD OF THE INVENTION:

The present invention relates to a liquid crystal compound, a liquid crystal composition containing the liquid crystal compound as a component and a liquid crystal display device with the liquid crystal composition sealed therein.

### DESCRIPTION OF THE PRIOR ART:

To obtain liquid crystal display devices having excellent response and wide viewing angle, it is necessary to reduce the viscosity and refractive anisotropy of a liquid crystal material used. For satisfying these requirements, phenylcyclohexane and phenyl cyclohexanecarboxylate liquid crystal materials obtained by introducing a cyclohexane ring into chemical structures of liquid crystal materials have been proposed. However, the phenyl cyclohexanecarboxylate liquid crystal compounds having an ester group between two six membered rings have insufficient physical properties such as viscosity and refractive anisotropy. For example, liquid crystal compounds represented by the general formula:

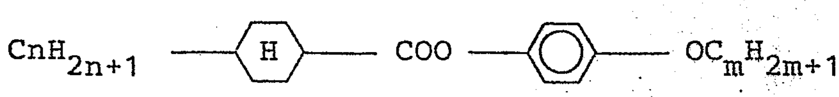

(hereinafter referred to as "conventional compounds") have too high viscosity and refractive anisotropy.

## SUMMARY OF THE INVENTION

An object of the present invention is to provide a new ester-type liquid crystal compound having low viscosity and refractive anisotropy. Another object of the invention is to provide a liquid crystal composition containing said liquid crystal compound as a component and a liquid crystal display device having a liquid crystal layer comprising the liquid crystal composition. The liquid crystal compound of the present invention is characterized by having the following molecular structure:

(a) the molecular skeleton has two or more six-membered rings,

(b) the respective six-membered rings are bonded directly to each other,

(c) a carbon atom in each of the terminal six-membered rings is bonded directly to an acyclic terminal group,

(d) one of the acyclic terminal groups bonded to the carbon atom of the six-membered ring is an alkyl group or an alkoxycarbonyl-free group bonding through an alkylene group, and

(e) the other acyclic terminal group bonded to the carbon atom in the six-membered ring has a carbonyl group and

a terminal alkoxyl group.

The typical liquid crystal compounds of the present invention are represented by the following formula:

$R_1-X-Y-R_2$

wherein $R_1$ is an alkyl group or a carboxyl-free acyclic group directly bonded to X through an alkylene group, $R_2$ is an acyclic group directly bonded to Y and having a carbonyl group and a terminal alkoxyl group, X is a six-membered ring selected from the group consisting of phenyl, cyclohexane and bicyclooctane rings and Y is a six-membered ring selected from the group consisting of phenyl and cyclohexane rings.

Liquid crystal compounds having an ester group as a central linkage as in the conventional compounds (namely, compounds in which the six-membered rings are not directly bonded to each other) inevitably have a high viscosity. After investigations, the inventors have found that the viscosity of the liquid crystal compounds can be reduced by directly bonding the six-membered rings to each other.

In a liquid crystal compound in which an oxygen atom is directly bonded to a six-membered ring such as a benzene ring, π-electrons of the benzene ring are conjugated with unshared electrons of the oxygen to become mobilized and thereby increase the dipole moment and dielectric constant. As a result, the viscosity and the refractive anisotropy are increased. After investigations made for the purpose of

introducing an ester group into a terminal of said compound to interpose a carbonyl group between the oxygen atom and the benzene ring, the inventors have succeeded in synthesizing an ester-type liquid crystal compound having a reduced dipole moment by preventing the electron mobilization. The present invention has been completed on the basis of these findings.

Introduction of alkoxycarbonyl groups into the ends of the both terminal acyclic groups gives rise to a smectic liquid crystal having an extremely high viscosity at ambient temperature. Therefore, if an alkoxycarbonyl-containing acyclic terminal group is required, such a group must be introduced into only one end of the compound in the present invention.

Also in the other acyclic terminal group, the direct bonding of oxygen to the six-membered ring is not preferred. Therefore, the acyclic terminal group in the liquid crystalline compound contains an alkyl or alkylene group according to the present invention.

The acyclic terminal groups are situated preferably in positions para with respect to the bonding position of the six-membered rings. With this arrangement, the liquid crystal molecule has a linear structure and a reduced viscosity.

The number of oxygen atoms is preferably 0 or 1 in the acyclic group defined in the above item (d) and preferably 2 in the acyclic group defined in the above item (e), because

if more oxygen atoms are present, the viscosity will be increased by the polarity of the oxygen atoms themselves in both cases.

The acyclic group as defined in the item (d) is bonded to a carbon atom in the six-membered ring through methylene group(s) and it may have a terminal alkoxyl group. The acyclic group is represented by, for example, the following formula:

$- (CH_2O)_xC_yH_{2y+1}$ ,

$- (CH_2)_xOC_yH_{2y+1}$ ,

$- (CH_2)_xO(CH_2)_zOC\ H_{2y+1}$ or

$- CH_2O(CH_2)_xOC_yH_{2y+1}$

wherein x and z are each an integer of 1 to 4 and y is an integer of 1 to 8.

The acyclic group defined in the item (e) is represented by, for example, the following formula:

$- COOR$ ,

$- (CH_2)_nCOOR$ ,

$- CO(CH_2)_nOR$ or

$- (CH_2)_mCO(CH_2)_nOR$

wherein R is an alkyl group and m and n are each an integer of 1 to 8. The number of carbon atoms in the acyclic group is preferably up to 13, since the viscosity is increased as the number of carbon atoms is increased.

The number of carbon atoms in the acyclic group as

defined in the item (d) is preferably up to 12 for the same reason as above.

The liquid crystal compound of the present invention has at least two six-membered rings preferably selected from the group consisting of phenyl, cyclohexane and bicyclooctane rings.

The liquid crystal compound of the present invention has a high chemical stability.

The liquid crystal compound of the present invention is obtained by, for example, the following process:

(a) 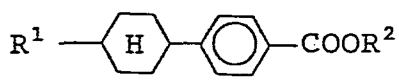

A compound of the formula: 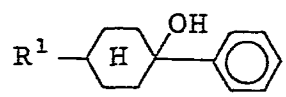 is obtained by a Gringnard reaction of 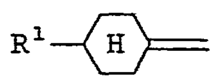 with 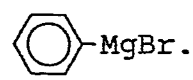. The obtained compound is dehydrated to obtain a compound of the formula: 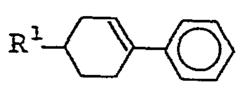, which is then hydrogenated to obtain a compound of the formula: 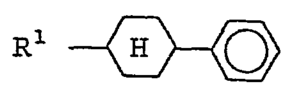.

This product is acetylated by a Friedel-Crafts reaction and the product is recrystallized from a suitable solvent to

obtain $R^1$ 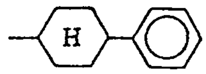 $COCH_3$. The trans isomer of this compound is converted into $R^1$ 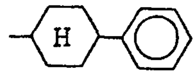 COOH by a haloform reaction. This compound is esterified with a suitable aliphatic alcohol ($R^2OH$) and sulfuric acid to obtain

$R^1$ 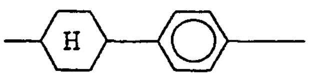 $COOR^2$ .

(b) $R^1$ 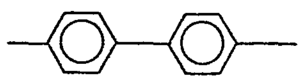 $COOR^2$

RCOCl and 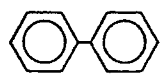 are subjected to a Friedel-Crafts reaction in the presence of $AlCl_3$ catalyst to obtain

RCO 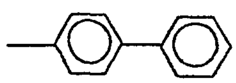 , which is then reduced with hydrazine to obtain $R^1$ 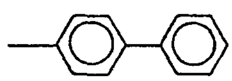 . Then, the acetylation, haloform reaction and esterification reaction are effected in the same manner as in item (a) to obtain $R^1$ 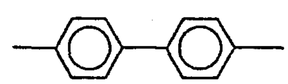 $COOR^2$.

(c) $R^1$ 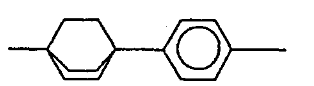 $COOR^2$

$R^1$ 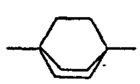 Br and 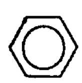 are subjected to a Friedel-Crafts

reaction to obtain 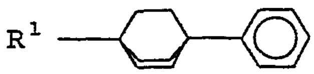 . This compound is subjected to the acetylation, haloform reaction and esterification reaction in the same manner as in item (a) to obtain 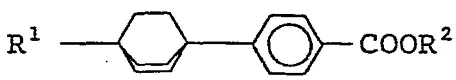 .

The liquid crystal composition of the present invention comprises the above-mentioned liquid crystal compound alone or a mixture thereof with other liquid crystal compounds and it may further contain additives such as a dichroic dye and an optically active substance. The proportion of the components may be determined without any limitation but the amount of the liquid crystal compound is preferably at least t wt.%. A TN display can be realized by using a liquid crystal compound having a positive dielectric anisotropy and a color display can be realized by using a liquid crystal compound having a negative dielectric anisotropy and a dichroic dye. Further, any sort of the display can be realized by suitably selecting additives.

A liquid crystal display device of the present invention comprises the liquid crystal composition of the present invention selaed in a liquid crystal cell comprising two opening substrates, a transparent electrode placed on each of the opposing surfaces of the substrates, an insulating and

orientation-controlling membrane placed on each of the opposing surfaces of the transparent electrodes which membrane acts to arrange adjacent liquid crystal molecules in a given direction on the substrate and a sealing member surrounding the two opposing substrates.

As described above in detail, the new estertype liquid crystal compounds of the present invention are effective in reducing the viscosity and refractive anisotropy and, therefore, the liquid crystal compositions containing said compounds are effective in forming liquid crystal display devices having excellent response and wide viewing angle.

## BRIEF DESCRIPTION OF THE DRAWINGS

Figs. 1 to 3 are infrared absorption spectra of the colorless liquid crystal compounds of the present invention.

## DETAILED DESCRIPTION OF THE PREFERRED EMBODIMENTS:

The following examples will further illustrate the present invention. The values of phase transition temperature vary slightly according to the determination method and purity. In the following example, the phase transition temperatures in parentheses indicate monotropic transition temperatures.

Example 1

Process for producing methyl 4-(trans-4-propylcyclohexyl)

benzoate (3ACl) (a liquid crystal compound of the above formula in which $R^1$ is $C_3H_7$ and $R^2$ is $CH_3$) and physical properties thereof:

(a) Small pieces of iodine were added to an ethereal suspension of 2.7 g (0.1 mol) of powdered magnesium and a small amount of 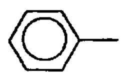 Br to initiate the reaction. Thereafter, 17.2 g (0.11 mol) of 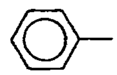 Br was added dropwise to the reaction mixture to reflux the ether mildly. After completion of the addition, the reaction mixture was aged at that temperature for 1 h and then cooled to 10 to 15°C. A solution of 14.0 g (0.1 mol) of $C_3H_7$ 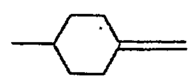 in ether was added dropwise thereto at that temperature and then the mixture was refluxed for 3 h. After cooling, the reaction mixture was poured into an aqueous solution of 6.5 g of $NH_4Cl$. The ethereal layer was washed with water and dried over $K_2CO_3$. Ether was distilled off. The residue was crude

$C_3H_7$ 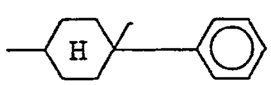 · $KHSO_4$ was added to this crude product and the mixture was heated to 100°C under stirring for 4 h

and distilled under reduced pressure to obtain $C_3H_7$ 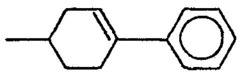 . The compound had a b.p. of 123 to 126°C (at 2 mmHg). The yield was 14.5 g (72%).

(b) About 1 g of Raney nickel W-4 was added to a solution of 20.0 g (0.1 mol) of $C_3H_7$ 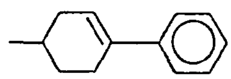 in ethanol and the hydrogenation reaction was carried out in an autoclave at room temperature under a hydrogen pressure of 50 kg/cm$^2$ for 3 h. Then, the Raney nickel was removed, ethanol was distilled off and the residue was distilled under reduced pressure to obtain $C_3H_7$ 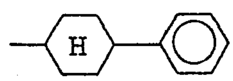 . This compound had a b.p. of 106 to 109°C (at 2 mmHg), Yield: 19.4 g (96%).

(c) 9.9 g (0.12 mol) of $CH_3COCl$ was added dropwise to a mixture of 20.2 g (0.1 mol) of $C_3H_7$ 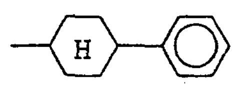 , 16 g (0.12 mol) of anhydrous $AlCl_3$ and 70 cc of $CH_2Cl_2$ under stirring at 0°C. After completion of the addition, the temperature was elevated to room temperature and the reaction mixture was aged for 3 h and poured into ice-water/hydro-

chloric acid. A $CH_2Cl_2$ layer was washed with water and dried. $CH_2Cl_2$ was distilled off. The residue was recrystallized from acetone to obtain 15.4 g (yield: 63%) of trans

$C_3H_7$ 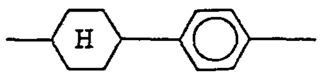 $COCH_3$. This compound was in the form of a smectic crystal at 45 to 54°C.

(d) An NaOBr solution comprising 62.5 g of NaOH, 69.7 g of bromine and 320 mℓ of water was added dropwise to a solution of 24.4 g (0.1 mol) of $C_3H_7$ 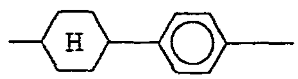 $COCH_3$ in dioxane under stirring at room temperature. After completion of the addition, the temperature was elevated slowly to 50°C. An aqueous $Na_2SO_3$ solution was added to the reaction mixture and the reaction was carried out at 90°C for 1 h. The reaction mixture was cooled and acidified with Hcl. The resulting crystals were recrystallized from ethanol to obtain 20.4 g (yield: 83%) of $C_3H_7$ 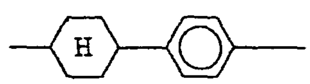 COOH.

(e) 4.92 g (0.02 mol) of $C_3H_7$ 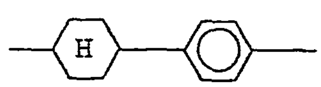 COOH, 60 mℓ of methanol and 1 mℓ of concentrated sulfuric acid were refluxed under stirring for 6 h. The reaction mixture was

poured in water and extracted with 20 mℓ of benzene twice. The benzene layer was washed with water. Benzene was distilled off and the residue was distilled under reduced pressure and then recrystallized from acetone to obtain 3.8 g (yield: 73%) of intended 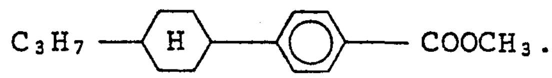.

The infrared spectrum of this compound is shown in Fig. 1. It is apparent from Fig. 1 that the compound obtained in this example had an absorption at 1720 $cm^{-1}$ due to the ester. In the mass a spectrum, a molecular ion peak was recognized at m/e 260. The results of elementary analysis of the resulting compound (C: 78.49%, O: 12.30% and H: 9.21%) coincided with the calculated values for $C_{17}H_{24}O_2$ (C: 78.46%, H: 9.23% and O: 12.31%). From a relationship between these results and the starting compounds, it was confirmed that the resulting compound was methyl 4-(trans-4-propylcyclohexyl)benzoate.

The compound had a phase transition temperature of 39.4°C (monotropic transition temp.: 33.7°C). It was thus a monotropic liquid crystal.

In the same manner as above, the following compounds can be obtained:

propyl 4-(trans-4-propylcyclohexyl)benzoate (3AC3) (a monotropic liquid crystal having a phase transition

temperature of 10.1°C and a monotropic transition temperature of -0.2°C), pentyl 4-(trans-4-propylcyclohexyl)benzoate (3AC5) a monotropic liquid crystal having a phase transition temperature of 22°C and a monotropic transition temperature of -8°C), methyl 4-(trans-4-pentylcyclohexyl)-benzoate (5AC1) (a monotropic liquid crystal having a phase transition temperature of 52°C and a monotropic transition temperature of 48.9°C), propyl 4-(trans-4-pentylcyclohexyl)benzoate (5AC3) (a liquid crystal having a phase transition temperature of 2.1 to 19.8°C), pentyl 4-(trans-4-pentylcyclohexyl)-benzoate (5AC5) (a liquid crystal having a phase transition temperature of 8.0 to 9.2°C), methyl 4-(trans-4-heptylcyclohexyl)benzoate (7AC1) (a liquid crystal having a phase transition temperature of 44.6 to 58.2°C), propyl 4-(trans-4-heptylcyclohexyl)benzoate (7AC3) (a liquid crystal having a phase transition temperature of 13.8 to 33.0°C), pentyl 4-(trans-4-heptylcyclohexyl)-benzoate (7AC5) (a liquid crystal having a phase transition temperature of 14.0 to 22.4°C), propyl 4-(trans-4-propylcyclohexyl)cyclohexanecarboxylate (a smectic liquid crystal having a phase transition temperature of 24 to 52°C), propyl 4-(trans-4-pentylcyclohexyl) phenylbenzoate (a smectic liquid crystal having a phase transition temperature of 43 to 190°C), pentyl 4-(trans-4-ethylcyclohexyl)benzoate, pentyl 4-(trans-4-butylcyclohexyl)-benzoate, pentyl 4-(trans-4-hexylcyclohexyl)benzoate,

pentyl 4-(trans-4-heptylcyclohexyl)benzoate and pentyl 4-(trans-4-octylcyclohexyl)benzoate.

Example 2

<u>Process for producing propyl 4-(4-pentylphenyl)benzoate (5AB3) (a liquid crystal compound of the above formula in which $R^1$ is $C_5H_{11}$ and $R^2$ is $C_3H_7$) and physical properties thereof:</u>

(a) 23.3 g (0.175 mol) of anhydrous $AlCl_3$ was added to a solution of 24.6 g (0.16 mol) of 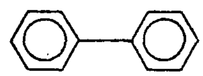 in $CH_2Cl_2$. 19.7 g (0.16 mol) of $C_4H_9COCl$ was added dropwise to the mixture under stirring at a temperature of 0°C or below. After stirring for 5 h, the reaction mixture was poured in Hcl/ice-water. The $CH_2Cl_2$ layer was washed with water and $CH_2Cl_2$ was distilled off. The residue was recrystallized from isopropyl alcohol to obtain 29 g (yield: 78%) of

$C_4H_9CO$ 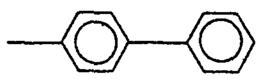 .

(b) 21.6 g (0.19 mol) of $C_4H_9CO$ 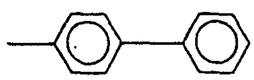 , 14.2 g (2.27 mol) of 80% $NH_2NH_2 \cdot H_2O$, 6.0 g (0.91 mol) of KOH and 120 mℓ of diethylene glycol were reacted at 220 to 230°C for 12 h. The reaction mixture was poured into water and

acidified with hydrochloric acid. After extraction with benzene, the benzene layer was washed with water. Benzene was distilled off and the residue was distilled under reduced pressure to obtain $C_5H_{11}$ 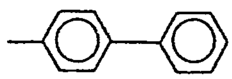 . The resulting compound had a b.p. of 181 to 183°C (at 9 mmHg). Yield was 16.7 g (82%).

(c) 5.5 g (0.07 mol) of $CH_3COCl$ was added dropwise to a mixture of 7.9 g (0.06 mol) of anhydrous $AlCl_3$ and 50 mℓ of $CH_2Cl_2$ under stirring at 10 to 15°C. After cooling to 0°C, 11.2 g (0.05 mol) of $C_5H_{11}$ 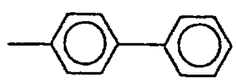 was added dropwise thereto at that temperature and the mixture was aged for 1 h. The reaction mixture was poured into hydrochloric acid/ice-water. The $CH_2Cl_2$ layer was washed with water. $CH_2Cl_2$ was distilled off and the residue was recrystallized from iropropyl alcohol to obtain 11.6 g (yield: 87%) of

$C_5H_{11}$ 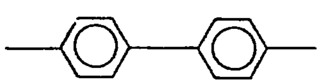 $COCH_3$ having a m.p. of 82.5°C.

(d) An NaOBr solution comprising 62.5 g of NaOH, 69.7 g of bromine and 320 mℓ of water was added dropwise to a solution solution of 26.6 g (0.1 mol) of $C_5H_{11}$ 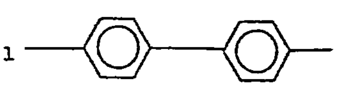 $COCH_3$

in dioxane under stirring at room temperature. After completion of the addition, the temperature was elevated slowly to 50°C. An aqueous $Na_2SO_3$ solution was added to the reaction mixture and the reaction was carried out at 90°C for 1 h. After cooling, the reaction mixture was acidified with HCl. The resulting crystals were recrystallized from ethanol to obtain 21 g (yield: 78%) of $C_5H_{11}$ 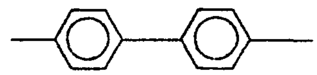 COOH.

(e) 5.3 g (0.02 mol) of $C_5H_{11}$ 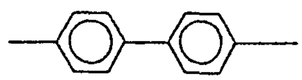 COOH, 50 mℓ $C_3H_7OH$ and 1 mℓ of conc. sulfuric acid were refluxed under stirring for 6 h to effect the reaction. Then, the reaction mixture was poured into water and extracted with benzene. The benzene layer was washed with water. Benzene was distilled off and the residue was distilled and then recrystallized from acetone to obtain 4.6 g (yield: 73%) of intended $C_5H_{11}$ 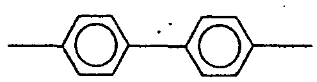 $COOC_3H_7$.

The infrared absorption spectrum of this compound is shown in Fig. 2. It is apparent from Fig. 2 that the compound obtained in this example had an absorption at 1715 $cm^{-1}$ due to the ester. In the mass spectrum, a molecular ion peak was recognized at m/e 310. The results of elementary

analysis of this compound (C: 81.26%, O: 10.33% and H: 8.41%) coincided with the calculated values for $C_{21}H_{26}O_2$ (C: 81.29, H: 8.39%, O: 10.32%). From these results and the starting compounds, it was confirmed that the resulting compound was propyl 4-(4-pentylphenyl)benzoate.

The compound had a phase transition temperature of 55.0 to 58.0°C.

In the same manner as above, the following compounds can be obtained:

propyl 4-(4-propylphenyl)benzoate (3AB3) (a monotropic liquid crystal having a phase transition temperature of 63°C and a monotropic transition temperature of 61°C), pentyl 4-(4-butylphenyl)benzoate, propyl 4-(4-hexylphenyl)benzoate, hexyl 4-(4-hexylphenyl)benzoate, propyl 4-(4-heptylphenyl)-benzoate, hexyl 4-(4-heptylphenyl)benzoate, ethyl 4-(4-octylphenyl)benzoate and pentyl 4-(4-octylphenyl)benzoate.

Example 3

Process for producing propyl 4-[4-propylbicyclo(2,2,2)-octyl]benzoate (3ABB3) (a liquid crystal compound of the above formula in which $R^1$ is $C_3H_7$ and $R^2$ is $C_3H_7$) and physical properties thereof:

(a) 2.9 g (0.018 mol) of anhydrous $FeCl_3$ was added in portions to a solution of 10 g (0.043 mol) of

$C_3H_7$ 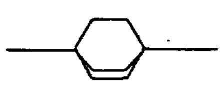 Br in benzene under stirring at room

temperature. The temperature was elevated slowly to 60°C. The reaction mixture was cooled and poured into dilute hydrochloric acid. The benzene layer was washed with water and benzene was distilled off. The residue was distilled under reduced pressure to obtain 9 g (91%) of

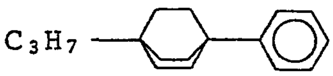. The resulting compound had a b.p. of 112 to 115°C (at 0.25 mmHg).

(b) 4.6 g (0.06 mol) of $CH_3COCl$ was added dropwise to a mixture of 7.87 g (0.06 mol) of anhydrous $AlCl_3$ and $CH_2Cl_2$ under stirring at 5°C. Then, a solution of 9 g (0.04 mol) of 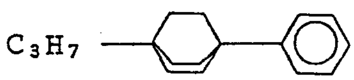 in $CH_2Cl_2$ was added dropwise thereto at a temperature of up to 5°C. The temperature was elevated slowly to room temperature and the reaction mixture was poured into dilute hydrochloric acid. The $CH_2Cl_2$ layer was washed with water. $CH_2Cl_2$ was distilled off and the residue was recrystallized from hexane to obtain 9.3 g (yield: 88%) of 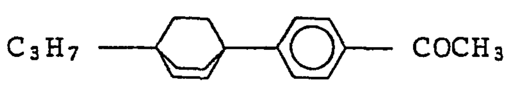. This compound had a m.p. of 90.0°C.

(c) 8.8 g (0.033 mol) of 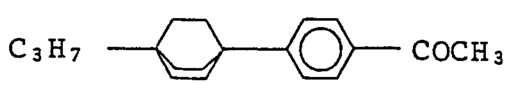 was

dissolved in dioxane. An NaOBr solution comprising 31.4 g of NaOH, 33.6 g of bromine and 160 mℓ of water was added dropwise to the solution under stirring at room temperature. After completion of the addition, the temperature was elevated slowly to 50°C and the reaction mixture was acidified with hydrochloric acid. After cooling, the resulting crystals were filtered and dried to obtain 6.5 g (yield: 72%) of 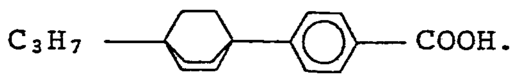.

(d) 5.42 g (0.02 mol) of 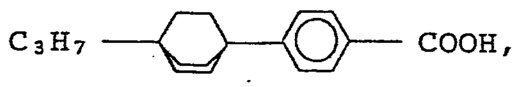, 50 mℓ of $C_3H_7OH$ and 1 mℓ of conc. sulfuric acid were reacted under stirring and under reflux for 6 h. The reaction mixture was poured into water and extracted with benzene. The benzene layer was washed with water. Benzene was distilled off and the residue was distilled and then recrystallized from acetone to obtain 4.7 g (yield: 75%) of intended

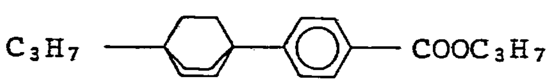.

The infrared absorption spectrum of this compound is shown in Fig. 3. It is apparent from Fig. 3 that the compound obtained in this example had an absorption at 1715 $cm^{-1}$ due to the ester. In the mass spectrum, a

molecular ion peak was recognized at m/e 314. The results of elementary analysis of this compound (C: 80.28%, O: 10.19% and H: 9.53%) coincided with the calculated values for $C_{21}H_{30}O_2$ (C: 80.26%, H: 9.55% and O: 10.19%). From these results and the starting compounds, it was confirmed that the resulting compound was propyl 4-[4-propylbicyclo (2,2,2)-octyl] benzoate.

The compound was in the form of liquid crystals having a phase transition temperature of 83.0 to 90.0°C.

In the same manner as above, the following compounds can be obtained:

methyl 4-[4-propylbicyclo(2,2,2)octyl] benzoate (3ABB1) (a monotropic liquid crystal having a phase transition temperature of 110°C and a monotropic transition temperature of 91°C), pentyl 4-[4-propylbicyclo(2,2,2)octyl] benzoate (3ABB5) (a monotropic liquid crystal having a phase transition temperature of 69°C and a monotropic transition temperature of 51°C), pentyl[4-(4-butylbicyclo(2,2,2)octyl]-benzoate, propyl 4-[4-pentylbicyclo(2,2,2)octyl] benzoate, pentyl 4-[4-pentylbicyclo(2,2,2)octyl] benzoate, propyl 4-[4-hexylbicyclo(2,2,2)octyl] benzoate, propyl 4-[4-heptylbicyclo-(2,2,2)octyl] benzoate and pentyl 4-[4-octylbicyclo(2,2,2) octyl)benzoate.

Example 4

<u>Process for producing a liquid crystal compound of the</u>

formula: 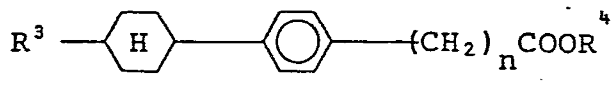, wherein n is an integer of 1 to 8:

A liquid crystalline compound of the formula:

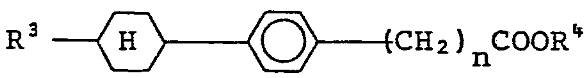 is produced in the same manner as in the above-mentioned examples. For example,

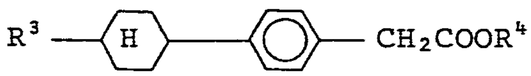 is obtained by synthesizing

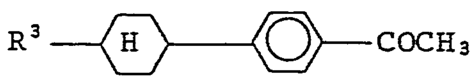 in the same manner as in Example 1 (a) and oxidizing the same with a suitable aliphatic alcohol ($R^2OH$), Tl $(NO_3)_3$ and 70% $HClO_4$ to obtain an intended

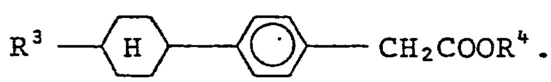.

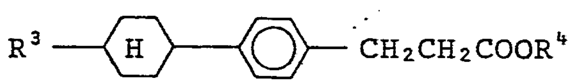 is obtained by synthesizing

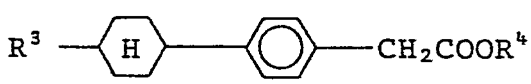 in the same manner as above, reducing the same with $LiAlH_4$ to obtain 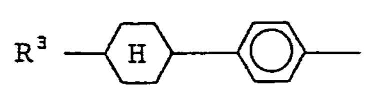

$CH_2CH_2OH$, converting this product with $SOCl_2$ into

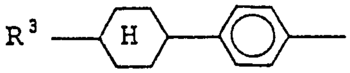 $CH_2CH_2C\ell$, converting the same with NaCN into 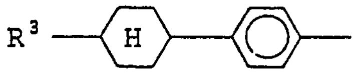 $CH_2CH_2CN$, hydrolyzing the same with KOH to obtain 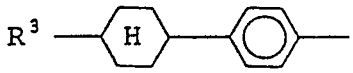 $CH_2CH_2COOH$ and esterifying the resulting compound with a suitable aliphatic alcohol ($R^4OH$) and sulfuric acid to obtain the intended

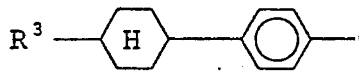 $CH_2CH_2COOR^4$.

In the same manner as above, the following compounds are obtained: methyl 4-(trans-4-propylcyclohexyl)-phenylacetate, propyl 4-(trans-4-propylcyclohexyl)-phenylacetate, pentyl 4-(trans-4-propylcyclohexyl)-phenylacetate, methyl 4-(trans-4-pentylcyclohexyl)-phenylacetate, propyl 4-(trans-4-pentylcyclohexyl)-phenylacetate, pentyl 4-(trans-4-pentylcyclohexyl)-phenylacetate, methyl 4-(trans-4-heptylcyclohexyl)-phenylacetate, propyl 4-(trans-4-heptylcyclohexyl)-phenylacetate, methyl 4-(trans-4-propylcyclohexyl)-phenylpropionate, propyl 4-(trans-4-propylcyclohexyl)-phenylpropionate, pentyl 4-(trans-4-propylcyclohexyl)-phenylpropionate, methyl 4-(trans-4-pentylcyclohexyl)-phenylpropionate, propyl 4-(trans-4-pentylcyclohexyl)-phenylpropionate, pentyl 4-(trans-4-pentylcyclohexyl)-phenylpropionate, methyl 4-(trans-4-pentylcyclohexyl)-phenylpropionate and propyl 4-(trans-4-pentylcyclohexyl)-phenylpropionate.

The samples thus obtained in the above-mentioned examples were added to colorless nematic liquid crystal compositions as shown in Table 1. The liquid crystal temperature ranges, viscosities and refractive anisotropies of the obtained colorless liquid crystal compositions are shown in Table 2 in comparison with the physical properties of the sample-free composition (i.e. a matrix liquid crystal alone) and those of compositions containing conventional liquid crystal compounds in place of the liquid crystal compounds of the present invention.

Table 1

| | | |
|---|---|---|
| Matrix liquid crystal | 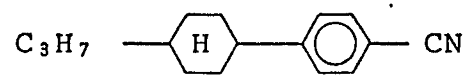 | 34 wt.% |
| | 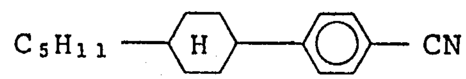 | 34 wt.% |
| | 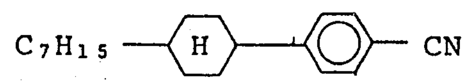 | 20 wt.% |
| | 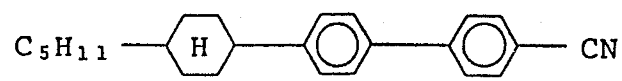 | 12 wt.% |

Table 2

| Symbol | Structural formula | Amount (mol%) | Liquid crystal temp. range (°C) | Vis-cosity (cp) (20°C) | Refrac-tive aniso-tropy (20°C) |
|---|---|---|---|---|---|
| 3AC1 | 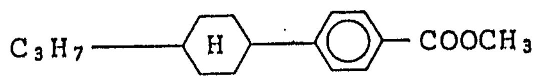 | 10 | 0∿66 | 22 | 0.0801 |
| 3AC1 | 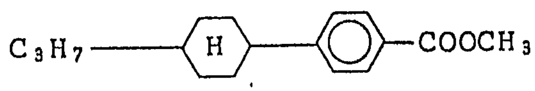 | 20 | 1∿63 | 22 | 0.0794 |
| 3AC1 | 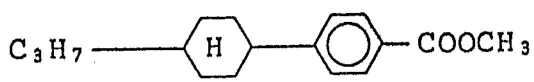 | 30 | 2∿59 | 21 | 0.0787 |
| 3AC5 | 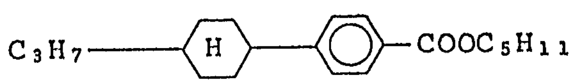 | 20 | -2∿54 | 22 | 0.0795 |

| | | | | | |
|---|---|---|---|---|---|
| 3AC3 | 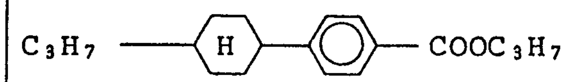 | 10 | -3∿64 | 21 | 0.0802 |
| 5AC1 | 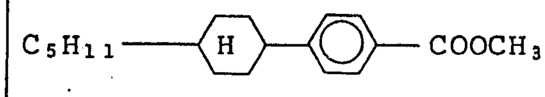 | 20 | 2∿66 | 21 | 0.0796 |
| 5AC3 | 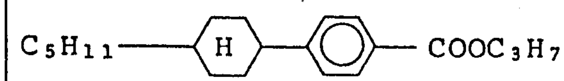 | 30 | 1∿55 | 21 | 0.0787 |
| 5AC5 | 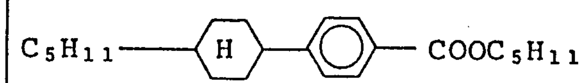 | 10 | -1∿64 | 22 | 0.0805 |
| 7AC1 | 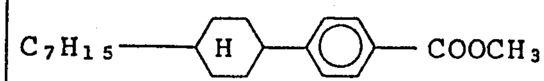 | 30 | 3∿63 | 22 | 0.0788 |

| | | | | | |
|---|---|---|---|---|---|
| 3AC3<br>5AC3 | 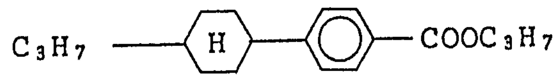<br>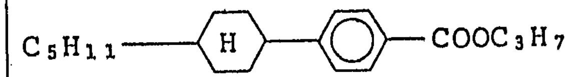<br>(equimolar) | 20 | -18∿58 | 22 | 0.0793 |
| 5A3<br>7AC3 | 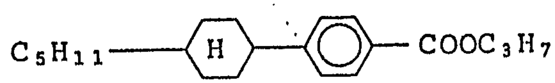<br>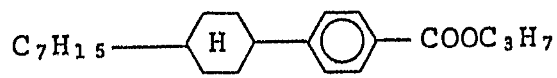<br>(equimolar) | 30 | -20∿57 | 23 | 0.0787 |
| 3AC1<br>7AC1 | 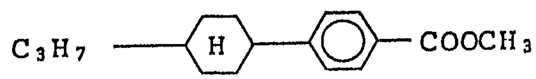<br>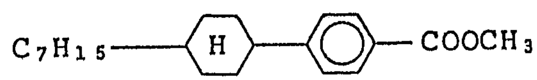<br>(equimolar) | 10 | -8∿68 | 22 | 0.0803 |

| | | | | | |
|---|---|---|---|---|---|
| 5AB3 | 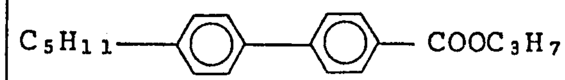 | 20 | 2∿68 | 25 | 0.0823 |
| 3AB5 | 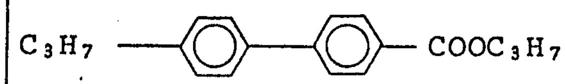 | 10 | 2∿69 | 25 | 0.0846 |
| 3ABB1 | 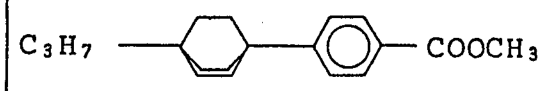 | 10 | 3∿72 | 26 | 0.0821 |
| 3ABB3 | 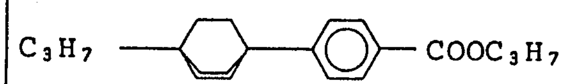 | 20 | 4∿74 | 26 | 0.0819 |
| 3ABB5 | 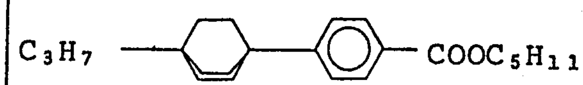 | 30 | 3∿64 | 26 | 0.0811 |

| | | | | | |
|---|---|---|---|---|---|
| Comp. Ex. | Matrix liquid crystal | - | -2∿71 | 29 | 0.0958 |
| | 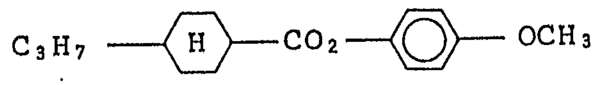 | 10 | 2∿69 | 30 | 0.0904 |
| | 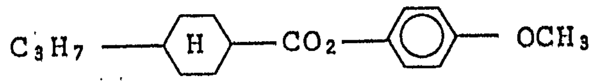 | 20 | 3∿68 | 31 | 0.0902 |
| | 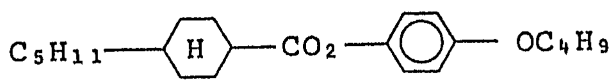 | 30 | 4∿73 | 32 | 0.0900 |
| | 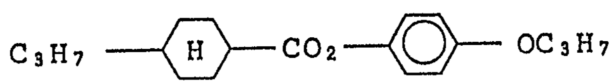 | 10 | 2∿69 | 31 | 0.0904 |

| | | | | | |
|---|---|---|---|---|---|
| | 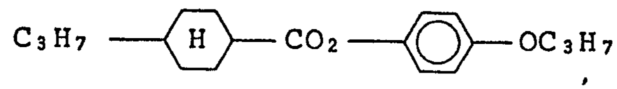 | 20 | 4∿68 | 32 | 0.0901 |
| | 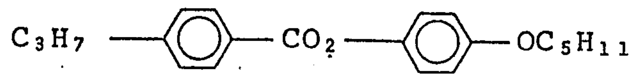 | 20 | 3∿65 | 34 | 0.0938 |
| | 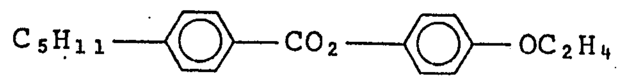 | 30 | 4∿67 | 35 | 0.0943 |
| | 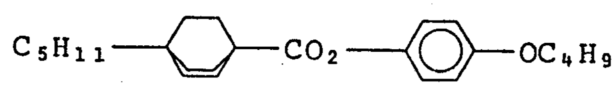 | 10 | 3∿73 | 35 | 0.0924 |
| | 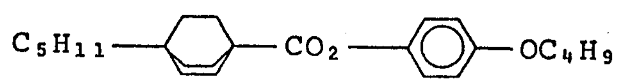 | 20 | 4∿76 | 37 | 0.0918 |

It is apparent from Table 2 that the liquid crystal compositions containing the liquid crystal compounds of the present invention are effective in reducing the viscosity and refractive anisotropy. On the other hand, the ester-type liquid crystal compounds in the comparative examples shown in Table 2 have defects that they increase the viscosity and refractive anisotropy remarkably.

CLAIMS:

1. A liquid crystal compound characterized by having the following molecular structure:

(a) the molecular skeleton has two or more six-membered rings,

(b) the respective six-membered rings are bonded directly to each other,

(c) a carbon atom in each of the terminal six-membered rings is bonded directly with an acyclic terminal group,

(d) one of the acyclic terminal groups bonded to the carbon atom of the six-membered ring is an alkyl group or an alkoxycarbonyl-free group bonding through an alkylene group, and

(e) the other acyclic terminal group bonded to the carbon atom in the six-membered ring has a carbonyl group and a terminal alkoxyl group.

2. A liquid crystal compound according to Claim 1

characterized in that each of the acyclic terminal groups is situated in a position para with respect to the bonding position of the six-membered rings.

3. A liquid crystal compound according to Claim 1 characterized in that the number of oxygen atoms contained in one of the acyclic terminal groups, i.e., the one defined in item (d) of Claim 1, is 0 or 1.

4. A liquid crystal compound according to Claim 1 characterized in that the number of oxygen atoms contained in the other acyclic terminal group, i.e., the one defined in item (e) of Claim 1, is 2.

5. A liquid crystal compound according to Claim 3 characterized in that said acyclic terminal group is bonded to the carbon atom in the six-membered ring through methylene group(s) and it has a terminal alkoxyl group.

6. A liquid crystal compound according to Claim 4 characterized in that the other acyclic terminal group is represented by the formula: $-COOR$, $-(CH_2)_nCOOR$, $-CO(CH_2)_nOR$ or $-(CH_2)_mCO(CH_2)OR$, wherein R represents an alkyl group and m and n represent each an integer of 1 to 8.

7. A liquid crystal compound according to Claim 6 characterized in that the acyclic terminal group of the above formula has up to 13 carbon atoms.

8. A liquid crystal compound according to Claim 1 characterized in that the six-membered ring is selected from

the group consisting of phenyl, cyclohexane and bicyclooctane rings.

9. A liquid crystal compound characterized by having the following general formula:

$$R_1-X-Y-R_2$$

wherein $R_1$ is an alkyl group or an alkoxycarbonyl-free acyclic group directly bonded to X through and alkylene group, $R_2$ is an acyclic group directly bonded to Y and having a carbonyl group and a terminal alkoxyl group, X is a six-membered ring selected from the group consisting of phenyl, cyclohexane and bicyclooctane rings, and Y is a six-membered ring selected from the group consisting of phenyl and cyclohexane rings.

10. A liquid crystal compound according to Claim 9 characterized in that the group $R_2$ contains a carboxyl group.

11. A liquid crystal compound according to Claim 9 characterized in that the groups $R_1$ and $R_2$ are situated in a position para with respect to the bonding position of X and Y.

12. A liquid crystal compound according to Claim 9 characterized in that the number of oxygen atoms in the group $R_1$ is 0 or 1.

13. A liquid crystal compound according to Claim 9 characterized in that the number of oxygen atoms in the group $R_2$ is 2.

14. A liquid crystal compound according to Claim 10 characterized in that $R_1$ is an alkyl group and $R_2$ is an alkoxycarbonyl group.

15. A liquid crystal composition characterized by containing a liquid crystal compound having the following molecular structure as a component:

(a) the molecular skeleton has two or more six-membered rings,

(b) the respective six-membered rings are bonded directly to each other,

(c) a carbon atom in each of the terminal six-membered rings is bonded directly to an acyclic terminal group,

(d) one of the acyclic terminal groups bonded to the carbon atom of the six-membered ring is an alkyl group or an alkoxycarbonyl-free group bonding through an alkylene group, and

(e) the other acyclic terminal group bonded to the carbon atom in the six-membered ring has a carbonyl group and a terminal alkoxyl group.

16. A liquid crystal composition according to Claim 15 characterized in that the amount of the liquid crystal compound is at least 5 wt.%.

17. A liquid crystal composition according to Claim 15 characterized in that each of the acyclic terminal groups is situated in a position para with respect to the bonding

position of the six-membered rings.

18. A liquid crystal composition according to Claim 15 characterized in that the number of oxygen atoms contained in one of the acyclic terminal groups, i.e., the one defined in item (d) of Claim 15, is 0 or 1.

19. A liquid crystal composition according to Claim 15 characterized in that the number of oxygen atoms contained in the other acyclic terminal group, i.e., the one defined in item (e) of Claim 15, is 2.

20. A liquid crystal composition according to Claim 18 characterized in that said acyclic terminal group is bonded to the carbon atom in the six-membered ring through methylene group(s) and it has a terminal alkoxyl group.

21. A liquid crystal composition according to Claim 19 characterized in that the other acyclic terminal group is represented by the formula:

$-COOR$, $-(CH_2)_nCOOR$, $-CO(CH_2)_nOR$ or $-(CH_2)_mCO(CH_2)_nOR$,

wherein R represents an alkyl group and m and n represent each an integer of 1 to 8.

22. A liquid crystal composition according to Claim 21 characterized in that the acyclic terminal group of the above formula has up to 13 carbon atoms.

23. A liquid crystal composition according to Claim 15 characterized in that the six-membered ring is selected from the group consisting of phenyl, cyclohexane and bicyclooctane

rings.

24. A liquid crystal display device characterized by comprising a liquid crystal composition containing as a component a liquid crystal compound having the following molecular structure:

(a) the molecular skeleton has two or more six-membered rings,

(b) the respective six-membered rings are bonded directly to each other,

(c) a carbon atom in each of the terminal six-membered rings is bonded directly to an acyclic terminal group,

(d) one of the acyclic terminal groups bonded to the carbon atom of the six-membered ring is an alkyl group or an alkoxycarbonyl-free group bonding through an alkylene group, and

(e) the other acyclic terminal group bonded to the carbon atom in the six-membered ring has a carbonyl group and a terminal alkoxyl group

which composition is sealed in a liquid crystal cell comprising two opposing substrates, a transparent electrode placed on each of the opposing surfaces of the substrates, an insulating and orientation-controlling membrane placed on each of the opposing surfaces of the transparent electrodes which membrane acts to arrange adjacent liquid crystal molecules in a given direction on the substrate surface and a sealing

member surrounding the two opposing substrates.

25. A liquid crystal display device according to Claim 24 characterized in that the liquid crystal composition contains at least 5 wt.% of the liquid crystal compound.

26. A liquid crystal display device according to Claim 24 characterized in that each of the acyclic terminal groups is situated in a position para with respect to the bonding position of the six-membered rings.

27. A liquid crystal display device according to Claim 24 characterized in that the number of oxygen atoms contained in one of the acyclic terminal groups, i.e., the one defined in item (d) of Claim 24, is 0 or 1.

28. A liquid crystal display device according to Claim 24 characterized in that the number of oxygen atoms contained in the other acyclic terminal group, i.e., the one defined in item (e) of Claim 24, is 2.

29. A liquid crystal display device according to Claim 27 characterized in that said acyclic terminal group is bonded to the carbon atom in the six-membered ring through methylene group(s) and it has a terminal alkoxyl group.

30. A liquid crystal display device according to Claim 28 characterized in that the other acyclic terminal group is represented by the formula:

$-COOR$, $-(CH_2)_nCOOR$, $-CO(CH_2)_nOR$ or $-(CH_2)_mCO(CH_2)_nOR$,

wherein R represents an alkyl group and m and n represent

each an integer of 1 to 8.

31. A liquid crystal display element according to Claim 30 characterized in that the acyclic terminal group of the above formula has up to 13 carbon atoms.

32. A liquid crystal display element according to Claim 24 characterized in that the six-membered ring is selected from the group consisting of phenyl, cyclohexane and bicyclooctane rings.

0118046

FIG. 1

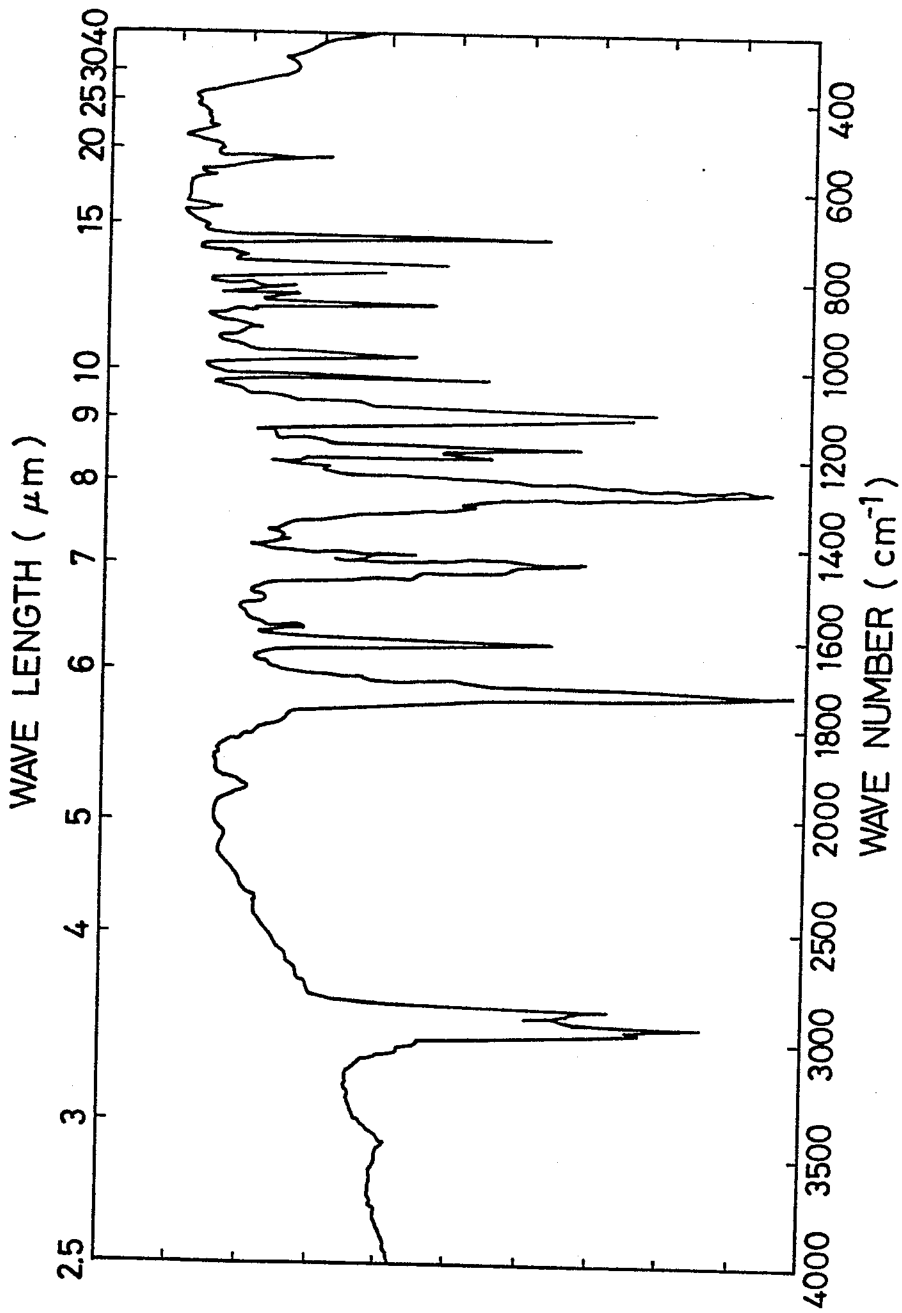

FIG. 2

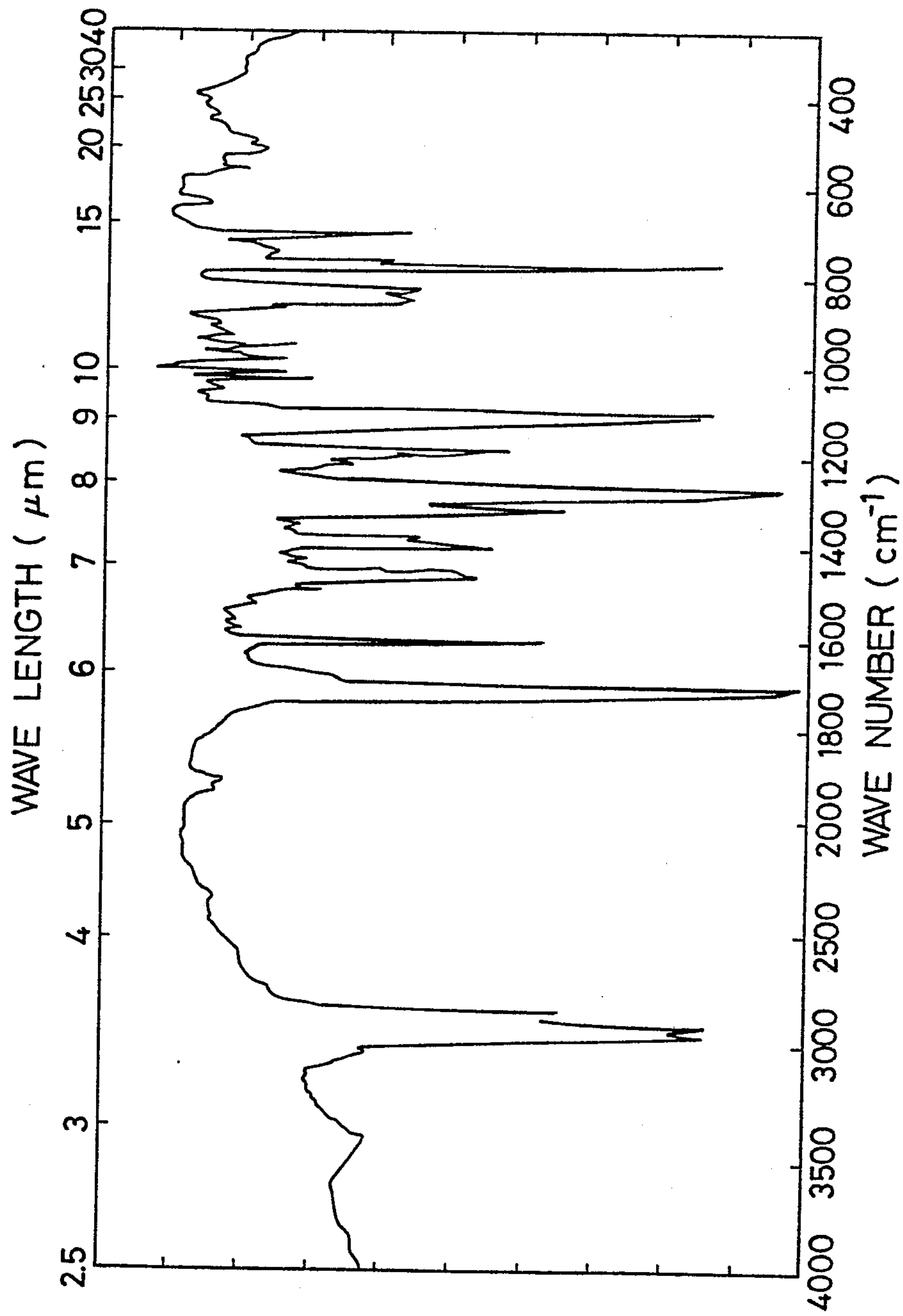
WAVE LENGTH ( μm )
2.5
3
4
5
6
7
8
9 10
15
20 25 30 40
WAVE NUMBER ( cm⁻¹ )
4000
3500
3000
2500
2000
1800
1600
1400
1200
1000
800
600
400

FIG. 3

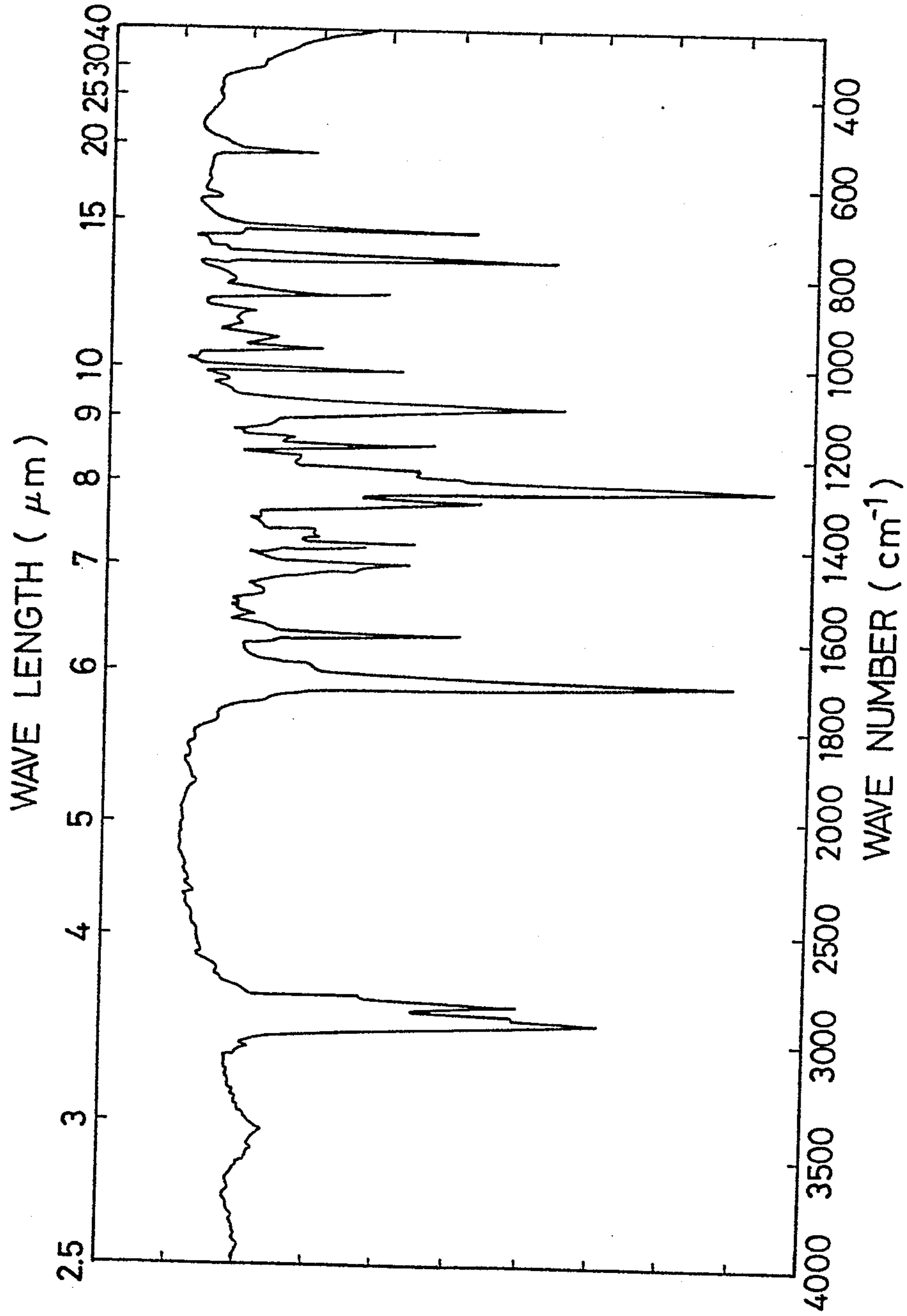
WAVE LENGTH ( μm )
2.5
3
4
5
6
7
8
9
10
15
20
25
30
40
WAVE NUMBER ( cm-1 )
4000
3500
3000
2500
2000
1800
1600
1400
1200
1000
800
600
400

European Patent Office

# EUROPEAN SEARCH REPORT

0118046 Application number

EP 84101232.1

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| X | PATENT ABSTRACTS OF JAPAN, unexamined applications, C field, vol. 6, no. 73, May 8, 1982<br>THE PATENT OFFICE JAPANESE GOVERNMENT<br>page 126 C 101<br>* Kokai-no. 57-11 944 (A) (CHISSO K.K.) *<br>-- | 1-4,6-14 | C 07 C 69/76<br>C 07 C 69/753<br>C 07 C 69/616<br>C 09 K 3/34<br>G 02 F 1/13 |
| A | GB - A - 2 071 649 (THE SECRETARY OF STATE FOR DEFENCE)<br>* Abstract *<br>-- | 1 | |
| P,X | EP - A1 - 0 087 032 (MERCK PATENT GESELLSCHAFT MIT BESCHRÄNKTER HAFTUNG)<br>* Claims 1,3-5; page 10, lines 17-19 *<br>-- | 1-4,6-19,21-28,30-32 | TECHNICAL FIELDS SEARCHED (Int. Cl. ³) |
| A | DE - A1 - 2 848 421 (TEMPLE UNIVERSITY)<br>* Claim 3 *<br>-- | 1 | C 07 C 69/00<br>C 09 K 3/00 |
| A | DE - A - 2 352 151 (MATSUSHITA ELECTRIC INDUSTRIAL CO., LTD.)<br>* Claim 12; pages 11,12; compounds 34-41 *<br>---- | 1 | |

The present search report has been drawn up for all claims

| Place of search | Date of completion of the search | Examiner |
|---|---|---|
| VIENNA | 03-05-1984 | HOFBAUER |

**CATEGORY OF CITED DOCUMENTS**

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503. 03.82